# FASCICULE DE BREVET EUROPEEN

(11) **EP 2 948 793 B1**
(45) Date de publication et mention de la délivrance du brevet: **21.08.2019**
(21) Numéro de dépôt: 14701941.8
(22) Date de dépôt: 16.01.2014
(51) Int. Cl.: G01T 1/161

(54) **DISPOSITIF DE RADIOLOGIE**
RADIOLOGIE-VORRICHTUNG
RADIOLOGY DEVICE

(30) Priorité: 23.01.2013 FR 1350578
(43) Date de publication de la demande: 02.12.2015
(73) Titulaire: A-G M Solutions B.V., 2676 LE Maasdijk (NL)
(72) Inventeur: GRAY, David, F-92160 Antony (FR)
(74) Mandataire: IPAZ
(86) Numéro de dépôt international: PCT/EP2014/050811
(87) Numéro de publication internationale: WO 2014/114555

(56) Documents cités:
- EP-A1- 0 168 604
- WO-A1-2006/038386
- US-A- 4 633 881
- US-A- 4 682 604
- US-A- 5 647 363

## Description

### Domaine technique

La présente invention concerne un dispositif de radiologie.

Un tel dispositif permet à un utilisateur d'étudier un traitement d'un patient dégageant une forte radioactivité sans risque pour le personnel médical ou pour d'autres patients. Le domaine de l'invention est plus particulièrement mais de manière non limitative celui de la thérapie des cancers par administration de radionucléides.

### Etat de la technique antérieure

Une méthode de soin d'un cancer d'un patient est de soumettre ce patient à une haute activité de substance radioactive, par administration de radionucléides, dans le but de réduire ou d'éliminer les cellules tumorales.

Compte tenu de la forte radioactivité d'un patient soumis à un tel traitement, deux options sont alors possibles :
- Soit d'étudier l'état du patient ; compte tenu de la forte radioactivité du patient, cela pose un problème pour la radioprotection d'une part du personnel médical en contact avec le patient et d'autre part des autres patients susceptibles de fréquenter la salle contenant le matériel dédié (par exemple « PET scan» ou scanner TEP pour « tomographie par émission de positons » en français)
- Soit placer le patient dans une chambre d'isolation, en l'isolant du personnel médical et en l'isolant des salles pouvant être partagées avec d'autres patients, jusqu'à ce que son niveau de radioactivité tombe en dessous d'un certain seuil, ce qui pose un problème du suivi du traitement et du patient.

Le but de l'invention est de proposer un dispositif permettant de résoudre ces deux problèmes.

Les documents WO2006038386 A1 et US4682604 divulguent des dispositifs de radiologie selon le préambule de la revendication 1.

### Exposé de l'invention

Cet objectif est atteint avec un dispositif de radiologie pour dosimétrie selon la revendication 1.

Les moyens de fixation peuvent comprendre un vêtement agencé pour accueillir au moins un des capteurs.

Le dispositif ne comprend de préférence que des capteurs de rayonnement gamma, et aucun détecteur de rayons X.

Les moyens de transmission et l'au moins une batterie sont de préférence regroupés dans un boîtier relié électriquement à chaque capteur par une liaison filaire agencée pour l'alimentation électrique de chaque capteur par l'au moins une batterie et pour le transfert des données vers les moyens de transmission.

Le dispositif selon l'invention peut comprendre en outre des moyens de mesure d'un rythme cardiaque et/ou des moyens de mesure d'un rythme respiratoire de l'utilisateur et des moyens pour synchroniser l'acquisition des données à partir de chaque capteur de rayonnement gamma avec le rythme cardiaque et/ou le rythme respiratoire.

### Description des figures et modes de réalisation

D'autres avantages et particularités de l'invention apparaîtront à la lecture de la description détaillée de mises en oeuvre et de modes de réalisation nullement limitatifs, et des dessins annexés suivants :
- la figure 1 illustre un mode de réalisation préféré de dispositif selon l'invention porté par un utilisateur humain 7 (aussi appelé le patient),
- la figure 2 illustre un capteur 2 du dispositif 1 selon l'invention de la figure 1, porté par son électronique d'acquisition 4 associée,
- la figure 3 illustre une électronique d'acquisition 4 d'un capteur 2 du dispositif 1 selon l'invention de la figure 1,
- la figure 4 illustre de manière détaillée une première partie 41 d'une électronique d'acquisition 4 d'un capteur 2 du dispositif 1 selon l'invention de la figure 1, et
- la figure 5 illustre de manière détaillée une deuxième partie 42 d'une électronique d'acquisition 4 d'un capteur 2 du dispositif 1 selon l'invention de la figure 1.

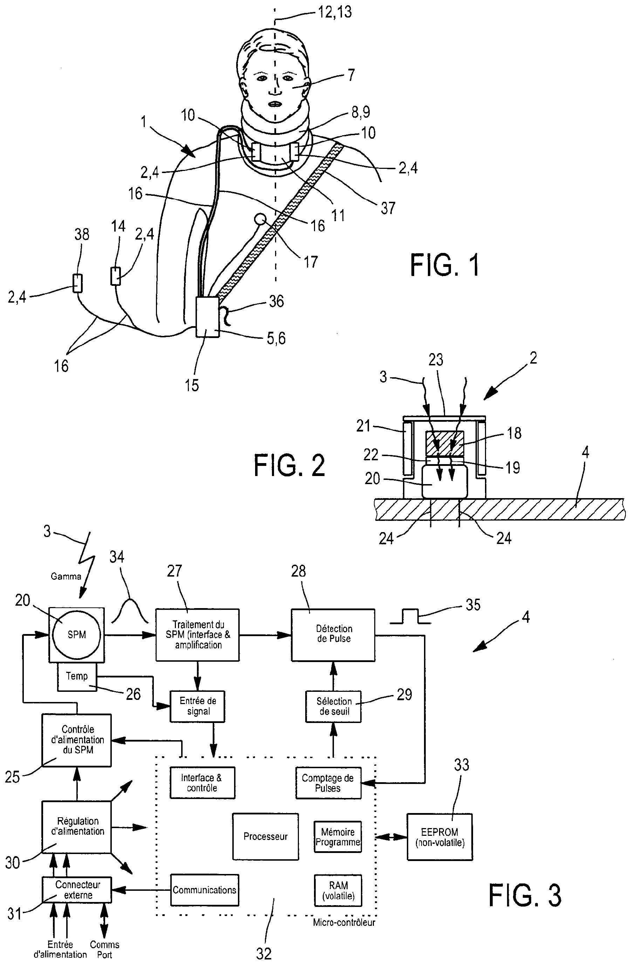

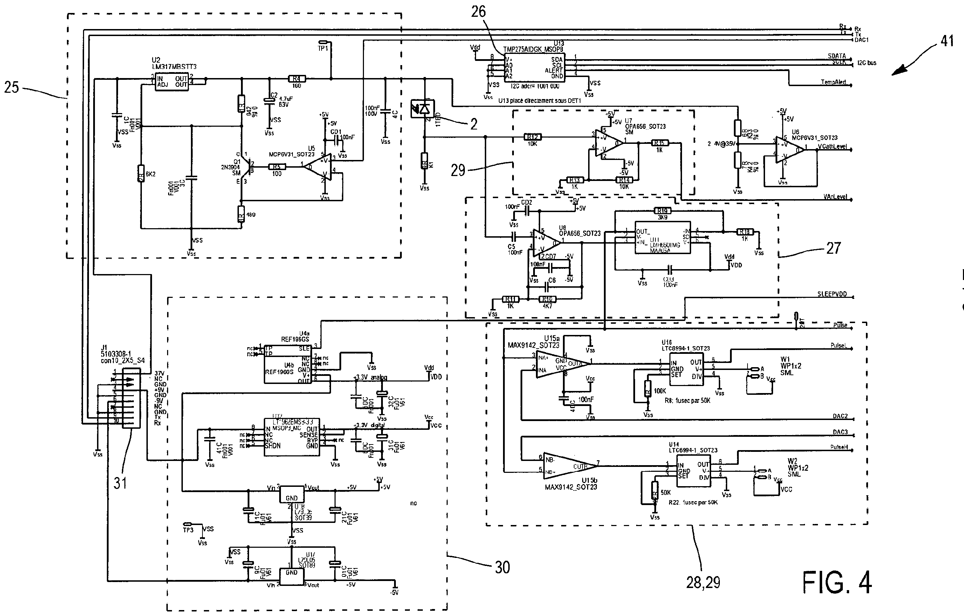

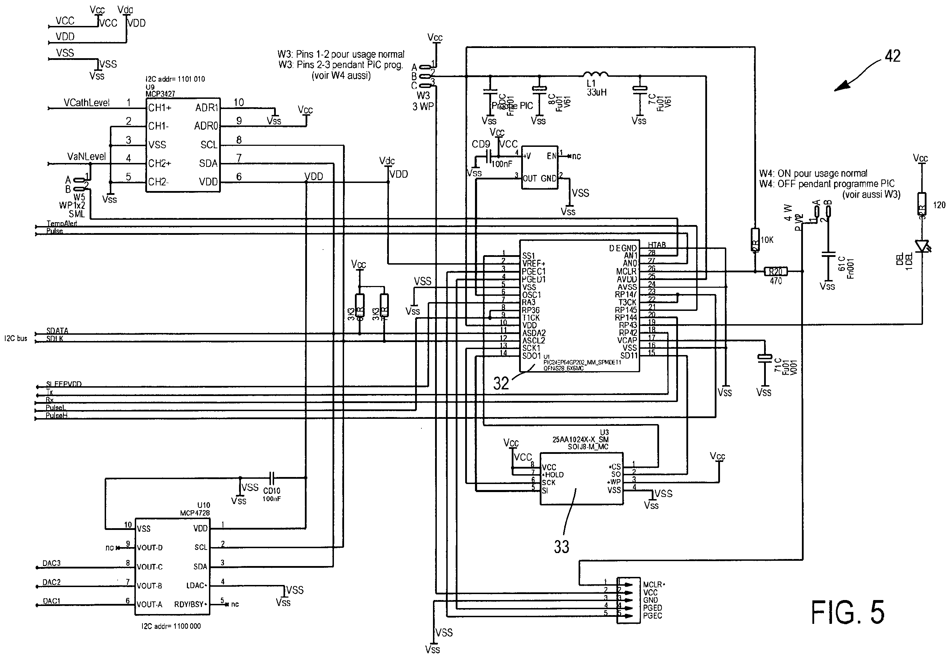

On va tout d'abord décrire, en référence aux figures 1 à 5, un mode de réalisation préféré de dispositif 1 de radiologie selon l'invention.

Par radiologie, on entend l'utilisation d'un rayonnement radioactif pour du comptage ou de la dosimétrie et/ou pour de l'imagerie.

Le dispositif 1 est un dispositif portable, c'est-à-dire destiné à être entièrement porté par un utilisateur 7.

Le dispositif 1 comprend au moins un capteur 2 de rayonnement gamma 3. Dans le cas du dispositif 1 illustré sur la figure 1, ce dispositif 1 comprend quatre capteurs 2.

Par rayonnement gamma 3, on entend de préférence un rayonnement électromagnétique de fréquence supérieure à 7·10¹⁸ Hertz, de préférence comprise entre 7·10¹⁸ Hertz et 3·10²² Hertz (ou bien de longueur d'onde inférieure à 10⁻¹¹ mètre, ou comprise entre 10⁻¹⁴ mètres et 10⁻¹¹ mètres). Chaque capteur est de préférence agencé pour détecter un rayonnement gamma d'énergie comprise entre 30 keV (Kilo électronvolts) et 600 keV, tel un rayonnement gamma correspondant à la Iodine-131 (364 keV)..

Dans le cas du dispositif 1, tous les capteurs 2 ont les mêmes caractéristiques techniques décrites par la suite. Chaque capteur 2 est par exemple un capteur de référence AGM SPM Sensor SSL-CsI-001.

Chaque capteur 2 de rayonnement gamma 3 comprend:
- un cristal de scintillation 18 (par exemple de type CsI(TI) Iode de Césium dopé au thallium) agencé pour transformer un rayonnement gamma 3 en au moins un nouveau photon 19 de longueur d'onde « visible » différente de (de préférence supérieure à) celle des photons du rayonnement gamma 3 (ces nouveaux photons 19 ayant une fréquence typique comprise entre 4,3·10¹⁴ Hertz et 9,4·10¹⁴ Hertz, ou une longueur d'onde comprise entre 320 nanomètres et 700 nanomètres), et
- un détecteur ou photomultiplicateur 20 (de préférence de type SiPM (pour « Silicon photomultiplier » en anglais ou « photomultiplicateur à silicium » en français) par exemple de référence SensL MicroSL30035-X13 ou Hamamatsu S10931-050P) agencé pour capter chaque nouveau photon 19 en provenance du cristal 18 et générer, en fonction des photons 19 captés, un signal électrique correspondant à une détection de rayonnement gamma par le capteur 2.

Le cristal 18 et le photomultiplicateur 20 de chaque capteur 2 sont montés à l'intérieur d'un conduit d'un collimateur 21 de préférence composé au moins en partie d'Aluminium et/ou de Tungstène.

Le cristal 18 et le photomultiplicateur 20 sont reliés optiquement et en contact avec une couche de gel optique 22 (par exemple de référence NyoGel OC-431A-LVP ou OC-462 de chez Neyco) de même indice optique que le cristal 18.

Le cristal 18 est de préférence précédé d'un filtre 23 ne laissant passer que les rayonnements gamma 3 de l'extérieur du dispositif 1 vers le cristal 18 (ou du moins, bloquant la lumière de longueur d'onde « visible » par le photomultiplicateur 20).

Le dispositif 1 comprend en outre des moyens d'acquisition 4, 41, 42 agencés pour acquérir des données à partir de chaque capteur 2 de rayonnement gamma 3.

Les moyens d'acquisition 4, 41, 42 comprennent une électronique d'acquisition 4 (typiquement une carte électronique) propre à chaque capteur 2 de rayonnement gamma et solidaire de ce capteur 2.

Le photomultiplicateur 20 de chaque capteur 2 est monté (soudure des électrodes 24 du photomultiplicateur 20 de ce capteur 2) sur la carte de l'électronique d'acquisition 4 associée à ce capteur 2.

Au sein de chaque électronique d'acquisition 4, un bloc électronique 27 de traitement (interface et amplification) est agencé pour mettre en forme un signal 34 (de forme quelconque, typiquement un pulse courbe) généré par le capteur 2 associé à cette électronique d'acquisition 4. Un bloc électronique 28 de détection de pulse est agencé pour générer un pulse (typiquement en créneau 35) si le signal 34, après traitement par le bloc 27, dépasse un certain seuil. Un bloc électronique 26 est agencé pour mesurer la température du photomultiplicateur 20. Ce bloc 26 comprend un détecteur de température de référence Texas Instruments TMP275. Un bloc électronique 29 de sélection de seuil est agencé pour ajuster ce seuil, de préférence en fonction de la température mesurée par le bloc 26, et est agencé pour pouvoir ne sélectionner qu'une partie du spectre des rayons gamma et pour calibrer le capteur 2. Un bloc électronique 25 est agencé pour contrôler l'alimentation électrique du photomultiplicateur 20, de préférence en fonction de la température mesurée par le bloc 26. Un micro-contrôleur 32 (de référence MicroChip PIC24EP64GP202) est agencé pour contrôler le tout. L'électronique d'acquisition 4 du capteur 2 comprend en outre une mémoire 33 typiquement de type EEPROM (pour «*Electrically-erasable programmable read-only memory* » en anglais, par exemple de référence MicroChip 25AA1024X), agencée pour mémoriser les données acquises à partir de ce capteur 2 par son électronique d'acquisition 4 associée. Un bloc électronique 30 d'alimentation est agencé pour distribuer et réguler l'énergie électrique pour l'ensemble de l'électronique d'acquisition 4 du capteur 2. L'électronique d'acquisition 4 du capteur comprend en outre un connecteur 31.

Pour chaque capteur 2, les données ainsi acquises par les moyens d'acquisition (i.e. par l'électronique d'acquisition 4 associée à ce capteur 2) comprennent typiquement :
- de préférence une mesure de rayonnement gamma (par exemple nombre de pulses 35) par unité de temps en fonction du temps (typiquement le capteur 2 va compter le nombre de pulsions 35 par seconde sur une durée de plusieurs secondes, de préférence chaque N minutes, avec N un nombre entier supérieur ou égal à 1) (par exemple plusieurs points de mesures, chaque point de mesure étant d'un format du type : 10 pulses 35 par seconde sur une période de temps T comprise entre t = 3 secondes et t = 6 secondes, avec un point de mesure toutes les 3 secondes ou toutes les minutes), ou
- une mesure de rayonnement gamma (par exemple nombre de pulses 35) en fonction du temps (par exemple plusieurs points de mesures, chaque point de mesure étant d'un format du type : 30 pulses 35 sur une période de temps T comprise entre t = 3 secondes et t = 6 secondes ; ou bien encore plusieurs points de mesures, deux points de mesure successifs étant d'un format du type : n^{ème} pulse détecté à t = 3 seconde, (n+1)^{ème} pulse détecté à t = 3,1 seconde)

Chaque capteur 2 et son électronique d'acquisition associée 4 sont agencés pour transformer ensemble un rayonnement gamma 3 en pulse électronique 35. Chaque électronique d'acquisition 4 est agencée pour compter le nombre de rayonnement gamma 3 qui frappe le capteur associé 2 de préférence dans une période T de temps prédéterminée, et pour stocker ce chiffre (dans la mémoire 33) pour plusieurs périodes consécutives. Ces données sont ensuite envoyées (via le connecteur 31) à une carte centrale (carte électronique des moyens de transmission 5).

Le dispositif 1 est agencé pour être intégralement porté par un utilisateur 7.

Le dispositif 1 est agencé pour ne pas nécessiter de liaison filaire du dispositif 1 vers l'extérieur du dispositif 1 lors de l'acquisition de données à partir d'au moins un capteur 2 par les moyens d'acquisition 4, 41, 42. Ainsi, le dispositif 1 selon l'invention permet de surveiller un patient 7 et son traitement, tout en lui permettant d'être libre de ses mouvements et de vivre normalement sans contrainte de mouvement. Le patient n'a plus besoin d'être dans une salle spécifique (telle une salle de PET scan) et peut être placé dans une salle telle qu'une salle d'isolation pour la sécurité du personnel médical et des autres patients.

Les moyens d'acquisition 4, 41, 42 sont de préférence agencés pour une acquisition continue de données de lecture dosimétrique de rayonnement gamma 3 sur une période de temps T, et pour mémoriser une telle acquisition sur plusieurs périodes T successives. Cela permet d'établir comment le patient 7 (typiquement sa thyroïde) réagit à l'administration des radionucléides thérapeutiques, de préférence alors que le patient 7 reste dans une salle d'isolation. En plus de la protection du personnel médical et des autres patients, cela permet un retour quantitatif sur l'absorption des radionucléides par le patient 7 et cela permet d'ajuster la thérapie en conséquence.

Le dispositif 1 comprend en outre des moyens de transmission 5 agencés pour transmettre vers l'extérieur du dispositif 1 les données acquises, et des moyens 36, 37 pour attacher les moyens de transmission 5 à une partie du corps de l'utilisateur 7.

Le dispositif 1 comprend en outre au moins une batterie 6 (par exemple 3 batteries en série de référence SAFT MP174565) agencée pour stocker de l'énergie électrique et alimenter électriquement chaque capteur 2, les moyens d'acquisition 4, 41, 42, et les moyens de transmission 5, et des moyens 36, 37 pour attacher l'au moins une batterie 6 à une partie du corps de l'utilisateur 7. L'au moins une batterie 6 est équipé d'une prise permettant de brancher cette au moins une batterie 6 à un cordon relié à une prise électrique pour recharger la batterie de manière occasionnelle, de préférence lorsque le dispositif 1 n'est pas porté ou utilisé par un utilisateur 7 pour acquérir des données à partir de capteur 2 de rayonnement gamma.

Les moyens de transmission 5 et l'au moins une batterie 6 sont regroupés dans un boîtier 15 et reliés électriquement à chaque capteur 2 (via le connecteur 31 de l'électronique d'acquisition 4 associée à chaque capteur 2) par une liaison filaire 16 agencée pour l'alimentation électrique de chaque capteur 2 par l'au moins une batterie 6 et pour le transfert vers les moyens de transmission 5 des données acquises à partir d'au moins un capteur 2 par les moyens d'acquisition 4. Le boitier 15 est équipé d'une bandoulière 37 et/ou d'une pince ou boucle 36 agencée pour fixer le boitier 15 à une ceinture de l'utilisateur 7.

Les moyens de transmission 5 comprennent des moyens de stockage (une mémoire par exemple de référence MicroChip 25AA1024X) agencés pour stocker dans le dispositif 1 les données acquises à partir d'au moins un capteur 2 par les moyens d'acquisition 4.

Les moyens de transmission 5 comprennent des moyens (par exemple un port USB pour connecter le dispositif 1 à un ordinateur ou un PC pour récupérer les données et les analyser) pour transmettre vers l'extérieur du dispositif 1 ces données par une liaison filaire, agencés pour transmettre ces données après leur acquisition par les moyens d'acquisition 4 et après leur stockage par les moyens de stockage. Ainsi, même lorsque le patient 7 est dans une salle d'isolation, le personnel médical est protégé de la radioactivité mais peut quand même accéder à la fin du traitement à des données décrivant l'évolution du traitement en fonction du temps, ces données étant bien plus complètes qu'un simple badge de dosimétrie photographique.

Les moyens de transmission 5 (comprenant typiquement un émetteur Wifi) sont agencés pour envoyer vers l'extérieur du dispositif 1 les données acquises à partir d'au moins un capteur 2 par les moyens d'acquisition 4 par une liaison sans fil (par exemple de type Wifi), de préférence au fur et à mesure de leur acquisition par les moyens d'acquisition 4, ou bien après leur stockage par les moyens de stockage. Ainsi, même lorsque le patient 7 est dans une salle d'isolation, le personnel médical est protégé de la radioactivité mais peut quand même suivre, dans une autre pièce, le traitement grâce aux moyens de transmission 5 agencés pour envoyer (de préférence en direct et non en différé) vers l'extérieur du dispositif 1 par une liaison sans fil (par exemple de type Wifi) les données acquises à partir d'au moins un capteur 2 par les moyens d'acquisition 4.

Le dispositif 1 comprend en outre (de préférence dans le boitier 15) un processeur agencé pour surveiller l'état de charge de l'au moins une batterie 6.

Le dispositif 1 comprend en outre des moyens de fixation 8 agencés pour fixer au moins un des capteurs 2 à une partie du corps de l'utilisateur 7. Les moyens de fixation 8 comprennent typiquement des moyens de serrage autour de cette partie du corps. On note que ces moyens de fixation 8 ne sont pas nécessairement présent pour tous les capteurs 2. Les moyens de fixation 8 ne sont pas communs pour tous les capteurs 2 mais sont distincts, de sorte que l'au moins un capteur 2 de rayonnement gamma 3 comprend plusieurs groupes 10, 14, 38 comprenant chacun au moins un capteur 2 de rayonnement gamma, chaque groupe 10, 14, 38 étant mobile par rapport aux autres groupes.

Dans le dispositif 1 les moyens de fixation 8 comprennent une minerve 9 pour un premier groupe 10 de deux capteurs 2 destinés à l'acquisition de rayonnement gamma 3 en provenance de la thyroïde de l'utilisateur 7 (un capteur 2 par lobe de la thyroïde).

De manière connue, une minerve (parfois aussi appelée collier cervical ou collet cervical) est un appareil placé autour du cou de l'utilisateur 7 et agencé pour maintenir la tête de l'utilisateur 7 dans une position fixe par rapport au cou de l'utilisateur 7, cette position fixe étant en générale droite et en extension. Une minerve est donc munie de moyens pour immobiliser les vertèbres cervicales (et donc le cou) de l'utilisateur 7.

Une minerve comprend en général un appui sternal et/ou un appui mandibulaire pour l'utilisateur 7.

Les deux capteurs 2 du premier groupe 10 sont disposés :
- sur ou dans une face avant 11 de la minerve 9, (sur une paroi intérieure ou extérieure de la minerve, ou dans la minerve) cette face avant 11 étant destinée à être positionnée :
   ∘ autour du cou de l'utilisateur 7, ce cou s'étendant longitudinalement le long d'un axe 12 de cou, et
   ∘ du côté de la face ventrale de ce cou, et
- symétriquement par rapport à un plan 13 de symétrie (perpendiculaire au plan de la figure 1) de sorte que l'axe 12 de cou appartienne à ce plan 13 de symétrie.

Un deuxième groupe 14 (dont chaque capteur n'est pas disposé sur la minerve et peut être déplacé indépendamment de la minerve) comprenant un unique capteur 2 est agencé pour servir de référence de mesure pour le premier groupe 10, c'est-à-dire pour mesurer le niveau ambiant de rayonnement de l'environnement de l'utilisateur. On note que ce deuxième groupe 14 n'est pas équipé par des moyens de fixation 8.

Un troisième groupe 38 comprenant un capteur 2 permet de faire des mesures sur une autre partie du corps par exemple sur la vessie du patient 7. La partie des moyens de fixation 8 (non illustrée) pour ce troisième groupe 38 comprend par exemple une surface autocollante ou un velcro ou une pince ou une boucle de ceinture, ou un vêtement (ceinture, veste, manche, gilet, culotte, casque, chapeau, pantalon, chaussette, chaussure, gant, etc...) agencé (par exemple grâce à une poche) pour accueillir un capteur 2.

De manière optionnelle, le dispositif 1 comprend en outre les éléments suivants (non illustrés sur les figures)) :
- tout d'abord :
   ∘ au moins un système de mesure d'un rythme cardiaque de l'utilisateur 7. Typiquement, chaque système de mesure d'un rythme cardiaque comprend un ECG (électrocardiogramme) par exemple de référence Lead-Lok A10. Chaque capteur 2 est équipé de son propre système de mesure d'un rythme cardiaque relié directement à l'électronique d'acquisition 4 de ce capteur 2 sans passer par le boitier 15 de la batterie 6 et des moyens de transmission 5, et/ou
   ∘ au moins un système 17 de mesure d'un rythme respiratoire de l'utilisateur 7. Typiquement, chaque système de mesure d'un rythme respiratoire comprend un système piézoélectrique de mesure de pression par exemple de référence Respironics CT2 P1823. Le dispositif 1 comprend un unique système 17 de mesure d'un rythme respiratoire, commun à tous les capteurs 2, et de préférence relié électriquement à chaque capteur 2 en passant par le boitier 15 de l'au moins une batterie 6 et des moyens de transmission 5,
- et en outre des moyens électroniques (à l'intérieur de chaque électronique d'acquisition 4) pour synchroniser l'acquisition des données à partir de chaque capteur 2 de rayonnement gamma 3 avec le rythme cardiaque et/ou le rythme respiratoire.

Ceci permet d'améliorer la précision des données acquises en supprimant certains artefacts de mesure.

Le dispositif 1 a un poids inférieur à un kilogramme.

On note que le dispositif 1 ne comprend que des capteurs de rayonnement gamma, et aucun détecteur de rayons X.

Bien sûr, l'invention n'est pas limitée aux exemples qui viennent d'être décrits et de nombreux aménagements peuvent être apportés à ces exemples sans sortir du cadre de l'invention.

En particulier on peut imaginer des variantes, combinables entre elles, du dispositif 1 :
- les moyens 36, 37 pour attacher l'au moins une batterie 6 et/ou pour attacher les moyens de transmission 5 peuvent être absents ; dans ce cas, on peut attacher l'au moins une batterie 6 et/ou les moyens de transmission 5 dans une poche ne faisant pas partie du dispositif 1 ou au moyen d'un scotch ou autocollant ou colle ne faisant pas partie du dispositif 1,
- au moins un des capteurs 2 peut comprendre plusieurs pixels (par exemple un ou plusieurs capteurs de référence SensL ArraySM-4), permettant de faire de l'imagerie (par exemple de la thyroïde dans le cas de la minerve), les données acquises permettant même de faire de l'imagerie en fonction du temps. Les données acquises à partir de cet au moins un des capteurs permettent de construire une image. Pour cela, le dispositif selon l'invention comprend (de préférence dans le boîtier 5 ou dans l'électronique 4 de cet au moins un des capteurs) des moyens (circuit électronique dédié) pour traiter les données acquises et construire une image sous la forme de données électroniques et/ou informatiques.

Bien entendu, les différentes caractéristiques, formes, variantes et modes de réalisation de l'invention peuvent être associées les unes avec les autres selon diverses combinaisons dans la mesure où elles ne sont pas incompatibles ou exclusives les unes des autres.

## Revendications

1. Dispositif (1) de radiologie pour dosimétrie, comprenant :
- au moins un capteur (2) de rayonnement gamma (3),
ledit dispositif comprenant en outre des moyens de fixation (8) agencés pour fixer au moins un des capteurs à une partie du corps de l'utilisateur, les moyens de fixation comprenant une minerve (9) pour au moins un des capteurs,
**caractérisé en ce qu'**il comprend en outre :
- des moyens d'acquisition (4, 41, 42) agencés pour acquérir des données dosimétriques à partir de chaque capteur de rayonnement gamma,
- des moyens de transmission (5) agencés pour transmettre vers l'extérieur du dispositif les données acquises,
- au moins une batterie (6) agencée pour stocker de l'énergie électrique et alimenter électriquement chaque capteur, les moyens d'acquisition, et les moyens de transmission,
les moyens d'acquisition étant agencés pour acquérir, à partir de chaque capteur (2) de rayonnement gamma :
- une mesure de rayonnement gamma par unité de temps en fonction du temps, ou
- une mesure de rayonnement gamma en fonction du temps,
le dispositif ayant un poids étant inférieur à un kilogramme
les moyens d'acquisition comprenant une électronique d'acquisition propre à chaque capteur de rayonnement gamma et solidaire de ce capteur
l'au moins un capteur de rayonnement gamma comprenant au moins un premier groupe (10) et un deuxième groupe (14), chaque groupe comprenant au moins un capteur de rayonnement gamma, les moyens de fixation n'étant pas commun pour le premier groupe (10) et le deuxième groupe (14), de sorte que :
- les moyens de fixation comprennent la minerve (9) pour le premier groupe (10), le premier groupe (10) comprenant deux capteurs disposés :
∘ sur une face avant (11) de la minerve, cette face avant étant destinée à être positionnée autour d'un cou s'étendant longitudinalement le long d'un axe (12) de cou et du côté de la face ventrale de ce cou, et
∘ symétriquement par rapport à un plan (13) de symétrie de sorte que l'axe de cou appartienne à ce plan de symétrie, et
- le deuxième groupe (14) est agencé pour servir de référence de mesure pour le premier groupe (10).

2. Dispositif selon l'une quelconque des revendications précédentes, **caractérisé en ce que** les moyens de transmission sont agencés pour envoyer vers l'extérieur du dispositif les données par une liaison sans fil, de préférence au fur et à mesure de leur acquisition par les moyens d'acquisition.

3. Dispositif selon l'une quelconque des revendications précédentes, **caractérisé en ce que** les moyens de transmission comprennent des moyens pour stocker les données dans le dispositif.

4. Dispositif selon la revendication 3, **caractérisé en ce que** les moyens de transmission comprennent des moyens pour transmettre vers l'extérieur du dispositif les données par une liaison filaire, agencés pour transmettre les données après leur acquisition par les moyens d'acquisition et leur stockage par les moyens de stockage.

5. Dispositif selon l'une quelconque des revendications précédentes, **caractérisé en ce que** les moyens de fixation comprennent en outre un vêtement agencé pour accueillir au moins un des capteurs.

6. Dispositif selon l'une quelconque des revendications précédentes, **caractérisé en ce que** les moyens de transmission et l'au moins une batterie sont regroupés dans un boîtier (15) relié électriquement à chaque capteur par une liaison filaire (16) agencée pour l'alimentation électrique de chaque capteur par l'au moins une batterie et pour le transfert des données vers les moyens de transmission.

7. Dispositif selon l'une quelconque des revendications précédentes, **caractérisé en ce qu'**il comprend en outre des moyens de mesure d'un rythme cardiaque et/ou des moyens (17) de mesure d'un rythme respiratoire de l'utilisateur et des moyens pour synchroniser l'acquisition des données à partir de chaque capteur de rayonnement gamma avec le rythme cardiaque et/ou le rythme respiratoire.

## Patentansprüche

1. Radiologievorrichtung (1) zur Dosimetrie, enthaltend:
- zumindest einen Sensor (2) für Gammastrahlung (3),
wobei die Vorrichtung ferner Befestigungseinrichtungen (8) enthält, die dazu vorgesehen sind, zumindest einen der Sensoren an einem Körperteil des Benutzers zu befestigen, wobei die Befestigungseinrichtungen eine Halskrause (9) für zumindest einen der Sensoren enthalten,
**dadurch gekennzeichnet, dass** sie ferner enthält:
- Erfassungseinrichtungen (4, 41, 42), die dazu vorgesehen sind, dosimetrische Daten von jedem Gammastrahlungssensor zu erfassen,
- Übertragungseinrichtungen (5), die dazu vorgesehen sind, die erfassten Daten nach außerhalb der Vorrichtung zu übertragen,
- zumindest eine Batterie (6), die dazu vorgesehen ist, elektrische Energie zu speichern und jeden Sensor, die Erfassungseinrichtungen und die Übertragungseinrichtungen elektrisch zu versorgen,
wobei die Erfassungseinrichtungen dazu vorgesehen sind, ausgehend von jedem Gammastrahlungssensor (2) zu erfassen:
- eine zeitabhängige Messung der Gammastrahlung pro Zeiteinheit oder
- eine zeitabhängige Messung der Gammastrahlung,
wobei die Vorrichtung ein Gewicht von weniger als einem Kilogramm hat,
die Erfassungseinrichtungen eine Erfassungselektronik enthalten, die jedem Gammastrahlungssensor eigen ist und fest mit diesem Sensor verbunden ist,
wobei der zumindest eine Gammastrahlungssensor zumindest eine erste Gruppe (10) und eine zweite Gruppe (14) umfasst, wobei jede Gruppe zumindest einen Gammastrahlungssensor umfasst, wobei die Befestigungseinrichtungen nicht für die erste Gruppe (10) und die zweite Gruppe (14) gemeinsam vorgesehen sind, so dass:
- die Befestigungseinrichtungen die Halskrause (9) für die erste Gruppe (10) enthalten, wobei die erste Gruppe (10) zwei Sensoren umfasst, die angeordnet sind
∘ an einer Vorderseite (11) der Halskrause, wobei diese Vorderseite dazu bestimmt ist, um einen Hals, der longitudinal entlang einer Halsachse (12) verläuft, und auf der ventralen Seite dieses Halses positioniert zu werden, und
∘ symmetrisch zu einer Symmetrieebene (13), so dass die Halsachse dieser Symmetrieebene angehört, und
- die zweite Gruppe (14) dazu vorgesehen ist, als Messreferenz für die erste Gruppe (10) zu dienen.

2. Vorrichtung nach dem vorangehenden Anspruch, **dadurch gekennzeichnet, dass**
die Übertragungseinrichtungen dazu vorgesehen sind, die Daten über eine drahtlose Verbindung nach außerhalb der Vorrichtung zu senden, vorzugsweise je nach deren Erfassung durch die Erfassungseinrichtungen.

3. Vorrichtung nach einem der vorangehenden Ansprüche, **dadurch gekennzeichnet, dass**
die Übertragungseinrichtungen Einrichtungen zum Speichern der Daten in der Vorrichtung enthalten.

4. Vorrichtung nach Anspruch 3, **dadurch gekennzeichnet, dass**
die Übertragungseinrichtungen Einrichtungen zum Übertragen der Daten über eine drahtgebundene Verbindung nach außerhalb der Vorrichtung enthalten, die dazu vorgesehen sind, die Daten nach deren Erfassung durch die Erfassungseinrichtungen und deren Speichern durch die Speichereinrichtungen zu übertragen.

5. Vorrichtung nach einem der vorangehenden Ansprüche, **dadurch gekennzeichnet, dass**
die Befestigungseinrichtungen ferner eine Auskleidung enthalten, die dazu vorgesehen ist, zumindest einen der Sensoren aufzunehmen.

6. Vorrichtung nach einem der vorangehenden Ansprüche, **dadurch gekennzeichnet, dass**
die Übertragungseinrichtungen und die zumindest eine Batterie in einem Gehäuse (15) zusammengefasst sind, das elektrisch mit jedem Sensor über eine drahtgebundene Verbindung (16) verbunden ist, die für die elektrische Versorgung eines jeden Sensors über die zumindest eine Batterie und für den Datentransfer zu den Übertragungseinrichtungen vorgesehen ist.

7. Vorrichtung nach einem der vorangehenden Ansprüche, **dadurch gekennzeichnet, dass**
sie ferner Messeinrichtungen zum Messen eines Herzrhythmus und/oder Messeinrichtungen (17) zum Messen eines Atmungsrhythmus des Benutzers und Einrichtungen zum Synchronisieren der Erfassung der Daten ausgehend von jedem Gammastrahlungssensor mit dem Herzrhythmus und/oder dem Atmungsrhythmus enthält.

## Claims

1. A radiology device (1) for dosimetry, comprising:
- at least one detector (2) of gamma radiation (3),
said device also comprising attachment means (8) arranged for attaching at least one of the detectors to a part of the user's body, the attachment means comprising a neck brace (9) for at least one of the detectors,
**characterized in that** it further comprises:
- acquisition means (4, 41, 42) arranged for acquiring dosimetric data from each gamma radiation detector,
- transmission means (5) arranged for transmitting the acquired data to outside the device,
- at least one battery (6) arranged for storing electrical power and supply electricity to each detector, to the acquisition means and to the transmission means,
the acquisition means being arranged for acquiring from each gamma detector (2):
- a gamma radiation measurement per unit of time as a function of time, or
- a gamma radiation measurement as a function of time,
the device having a weight being less than one kilogram,
the acquisition means comprising an acquisition electronic mean dedicated for each gamma radiation detector and integral with this detector;
the at least one detector of gamma radiation comprising at least one first group (10) and one second group (14), each group comprising at least one gamma radiation detector, the attachment means not being common for the first group (10) and the second group (14), so that:
- the attachment means comprise the neck brace (9) for the first group (10), the first group (10) comprising two detectors placed:
∘ on a front face (11) of the neck brace, this front face being intended to be positioned around a neck extending longitudinally along an axis (12) of the neck and on the side of the anterior face of this neck, and
∘ symmetrically with respect to a plane (13) of symmetry so that the axis of the neck forms part of this plane of symmetry;
- the second group (14) is arranged for acting as a measurement reference for the first group (10).

2. The device according to any one of the previous claims, **characterized in that** the transmission means are arranged for sending data to outside the device by a wireless link, preferably as they are acquired by the acquisition means

3. The device according to any one of the previous claims, **characterized in that** the transmission means comprise means for storing the data in the device.

4. The device according to claim 3, **characterized in that** the transmission means comprise means for transmitting data to outside the device by a wired connection, arranged for transmitting data after their acquisition by the acquisition means and their storage by the storage means.

5. The device according to any one of the previous claims, **characterized in that** the attachment means further comprise an item of clothing arranged to hold at least one of the detectors.

6. The device according to any one of the previous claims, **characterized in that** the transmission means and the at least one battery are grouped together in a case (15) electrically connected to each detector by a wired connection (16) arranged for supplying electricity to each detector via the at least one battery and for transferring data to the transmission means.

7. The device according to any one of the previous claims, **characterized in that** it also comprises means for measuring a heart rate and/or means (17) for measuring a respiration rate of the user and means for synchronizing the acquisition of data from each gamma radiation detector with the heart rate and/or the respiration rate.
